# EUROPEAN PATENT APPLICATION

(11) **EP 1 800 690 A2**
(43) Date of publication of application: **27.06.2007**
(21) Application number: 06017781.3
(22) Date of filing: 25.08.2006
(51) Int. Cl.: A61K 38/16, A61P 37/02

(54) **Use of fungal immunomodulatory protein**

(30) Priority: 23.09.2005 US 233364
(71) Applicant: Yeastern Biotech Co., Ltd., Taipei, Taiwan 221 (CN)
(72) Inventor: Chen, Tzu-Chih, Taipei 221 Taiwan (CN); Wang, Tsung-Tsan, Saint-Laurent Québeck H4L 5H6 (CA)
(74) Representative: Merkle, Gebhard

(57) **Abstract**

This present invention relates to the use of fungal immunomodulatory protein in immunotherapy, treating or activating natural killer cells, macrophage or increasing cytokines and serum antibody.

### Annex to the application documents - subsequently filed sequences listing

SEQUENCE LISTING

<110> Yeastern Biotech Co., Ltd.
<120> SEQ ID No:1
<160> 1
<170> PatentIn version 3.3
<210> 1
<211> 111
<212> PRT
<213> Ganoderma specisis
<221> PEPTIDE
<222> (1)..(111)
<400> 1

Met Ser Asp Thr Ala Leu Ile Phe Arg Leu Ala Trp Asp Val Lys Lys
1               5                   10                  15

Leu Ser Phe Asp Tyr Thr Pro Asn Trp Gly Arg Gly Asn Pro Asn Asn
            20                  25                  30

Phe Ile Asp Thr Val Thr Phe Pro Lys Val Leu Thr Asp Lys Ala Tyr
        35                  40                  45

Thr Tyr Arg Val Ala Val Ser Gly Arg Asn Leu Gly Val Lys Pro Ser
    50                  55                  60

Tyr Ala Val Glu Ser Asp Gly Ser Gln Lys Val Asn Phe Leu Glu Tyr
65                  70                  75                  80

Asn Ser Gly Tyr Gly Ile Ala Asp Thr Asn Thr Ile Gln Val Phe Val
                85                  90                  95

Val Asp Pro Asp Thr Asn Asn Asp Phe Ile Ile Ala Gln Trp Asn
            100                 105                 110

<110> Yeastern Biotech Co., Ltd.
<120> SEQ ID No:2

<160> 1
<170> PatentIn version 3.3
<210> 1
<211> 111
<212> PRT
<213> Ganoderma tsugae
<221> PEPTIDE
<222> (1)..(111)
<400> 1

Met Ser Asp Thr Ala Leu Ile Phe Arg Leu Ala Trp Asp Val Lys Lys
1               5                   10                  15

Leu Ser Phe Asp Tyr Thr Pro Asn Trp Gly Arg Gly Asn Pro Asn Asn
            20                  25                  30

Phe Ile Asp Thr Val Thr Phe Pro Lys Val Leu Thr Asp Lys Ala Tyr
        35                  40                  45

Thr Tyr Arg Val Ala Val Ser Gly Arg Asn Leu Gly Val Lys Pro Ser
    50                  55                  60

Tyr Ala Val Glu Ser Asp Gly Ser Gln Lys Val Asn Phe Leu Glu Tyr
65                  70                  75                  80

Asn Ser Gly Tyr Gly Ile Ala Asp Thr Asn Thr Ile Gln Val Phe Val
                85                  90                  95

Val Asp Pro Asp Thr Asn Asn Asp Phe Ile Ile Ala Gln Trp Asn
            100                 105                 110

<110> Yeastern Biotech Co., Ltd.
<120> SEQ ID No:3
<160> 1
<170> PatentIn version 3.3

<210> 1
<211> 114
<212> PRT
<213> Flammulina velutipes
<221> PEPTIDE
<222> (1)..(114)
<400> 1

Ser Ala Thr Ser Leu Thr Phe Gln Leu Ala Tyr Leu Val Lys Lys Ile
1               5                   10                  15

Asp Phe Asp Tyr Thr Pro Asn Trp Gly Arg Gly Thr Pro Ser Ser Tyr
            20                  25                  30

Ile Asp Asn Leu Thr Phe Pro Lys Val Leu Thr Asp Lys Lys Tyr Ser
        35                  40                  45

Tyr Arg Val Val Val Asn Gly Ser Asp Leu Gly Val Glu Ser Asn Phe
    50                  55                  60

Ala Val Thr Pro Ser Gly Gly Gln Thr Ile Asn Phe Leu Gln Tyr Asn
65                  70                  75                  80

Lys Gly Tyr Gly Val Ala Asp Thr Lys Thr Ile Gln Val Phe Val Val
                85                  90                  95

Ile Pro Asp Thr Gly Asn Ser Glu Glu Tyr Ile Ile Ala Glu Trp Lys
            100                 105                 110

Lys Thr

SEQUENCE LISTING

<110> Yeastern Biotech Co., Ltd.

<120> Use of Fungal Immunomodulatory Protein

<130> 0214-YB-EU-Me/fe

<140> 06017781.3/ EP06017781
<141> 2006-08-25

<150> US 11/233,364
<151> 2005-09-23

<160> 3

<170> PatentIn version 3.3

<210> 1
<211> 111
<212> PRT
<213> Ganoderma specisis

<220>
<221> PEPTIDE
<222> (1)..(111)

<400> 1

Met Ser Asp Thr Ala Leu Ile Phe Arg Leu Ala Trp Asp Val Lys Lys
1               5                   10                  15

Leu Ser Phe Asp Tyr Thr Pro Asn Trp Gly Arg Gly Asn Pro Asn Asn
            20                  25                  30

Phe Ile Asp Thr Val Thr Phe Pro Lys Val Leu Thr Asp Lys Ala Tyr
        35                  40                  45

Thr Tyr Arg Val Ala Val Ser Gly Arg Asn Leu Gly Val Lys Pro Ser
    50                  55                  60

Tyr Ala Val Glu Ser Asp Gly Ser Gln Lys Val Asn Phe Leu Glu Tyr
65                  70                  75                  80

Asn Ser Gly Tyr Gly Ile Ala Asp Thr Asn Thr Ile Gln Val Phe Val
                85                  90                  95

Val Asp Pro Asp Thr Asn Asn Asp Phe Ile Ile Ala Gln Trp Asn
            100                 105                 110

<210>  2
<211>  111
<212>  PRT
<213>  Ganoderma tsugae

<220>
<221>  PEPTIDE
<222>  (1)..(111)

<400>  2

Met Ser Asp Thr Ala Leu Ile Phe Arg Leu Ala Trp Asp Val Lys Lys
1               5                   10                  15

Leu Ser Phe Asp Tyr Thr Pro Asn Trp Gly Arg Gly Asn Pro Asn Asn
            20                  25                  30

Phe Ile Asp Thr Val Thr Phe Pro Lys Val Leu Thr Asp Lys Ala Tyr
        35                  40                  45

Thr Tyr Arg Val Ala Val Ser Gly Arg Asn Leu Gly Val Lys Pro Ser
    50                  55                  60

Tyr Ala Val Glu Ser Asp Gly Ser Gln Lys Val Asn Phe Leu Glu Tyr
65                  70                  75                  80

Asn Ser Gly Tyr Gly Ile Ala Asp Thr Asn Thr Ile Gln Val Phe Val
                85                  90                  95

Val Asp Pro Asp Thr Asn Asn Asp Phe Ile Ile Ala Gln Trp Asn
            100                 105                 110

<210>  3
<211>  114

<212>  PRT
<213>  Flammulina velutipes

<220>
<221>  PEPTIDE
<222>  (1)..(114)

<400>  3

Ser Ala Thr Ser Leu Thr Phe Gln Leu Ala Tyr Leu Val Lys Lys Ile
1               5                   10                  15

Asp Phe Asp Tyr Thr Pro Asn Trp Gly Arg Gly Thr Pro Ser Ser Tyr
            20                  25                  30

Ile Asp Asn Leu Thr Phe Pro Lys Val Leu Thr Asp Lys Lys Tyr Ser
        35                  40                  45

Tyr Arg Val Val Val Asn Gly Ser Asp Leu Gly Val Glu Ser Asn Phe
    50                  55                  60

Ala Val Thr Pro Ser Gly Gly Gln Thr Ile Asn Phe Leu Gln Tyr Asn
65                  70                  75                  80

Lys Gly Tyr Gly Val Ala Asp Thr Lys Thr Ile Gln Val Phe Val Val
                85                  90                  95

Ile Pro Asp Thr Gly Asn Ser Glu Glu Tyr Ile Ile Ala Glu Trp Lys
            100                 105                 110

Lys Thr

## Description

### Field of the Invention

This present invention relates to the use of fungal immunomodulatory proteins in immunotherapy.

### Description of the Prior Art

Ganoderma is a rare and valuable herb in Chinese medicine. It has been known in China for over 5,000 years as "Ling Zhi". There are a variety of ganodermas, including G *lucidum (red), G applanatum (brown), G tsugae (red), G sinense (black),* and *G. oregonense (dark brown).*

It has been known that Ling Zhi has anti-allergy (Chen H.Y et al., J. Med. Mycol. 1992; 33:505-512), hepatoprotective (Lin J.M. et al., Am J Chin Med. 1993; 21(1):59-69) and anti-tumor effects (Wasser SP, Crit Rev Immunol 1999. 19:65-96) and immune advantages (Kino, J Biol. Chem. 1989. 264(1) : 472-8). However, Ling Zhi is used restrictedly in the form of extract of raw material (Homer W.E. et al., Allergy 1993; 48:110-116) or small molecules (Kawagishi H., et al., Phytochemistry 1993; 32: 239-241).

Several proteins from edible fungi such as *Ganoderma Lucidium* (Ling zhi or Reishi), *Volvariella Volvacea* (Chinese Mushroom), *Flammulina Velutipes* (Golden needle mushroom) share similar amino acid sequences and immunomodulatory functions. These proteins were named fungal immunomodulatory proteins (FIPs) (Ko J.L., Eur. J. Biochem. 1995; 228:224-249).

Lin *et al.* have purified an immunomodulatory protein from the mycelium of *Ganoderma tsugae,* named FIP-*gts* (Lin, W.H., et al., J Biol Chem. 1997. 272, 20044-20048.). The FIP-*gts* found in the fruit body of *Ganoderma tsugae* has no immunomodulatory effect; only the protein found in the mycelium has the effect. After cloning, the DNA sequence of FIP-*gts* was found to be identical to the sequence of LZ-8 in *Ganoderma lucidium*. Both proteins exhibited the same immunoactivity, demonstrating that they are the same protein.

Analyzing the secondary structure with Garnier analysis, FIP-*gts* was predicted to have two α -helices, seven β -sheets and one β -turn. The molecular weight of FIP-*gts* was determined to be 13 kD using SDS-PAGE analysis. Connecting the amino acids with 20 µM glutaraldegyde (protein conjugate), FIP-*gts* was found to form a 26 kD homodimer.

In addition, three fungal proteins were found by Blast-formation stimulatory activity assay (BFSA). Except proteins found in *Ganoderma lucidium*, blood clotting proteins found in *Flammulina velutipes* and *Volvariella volvacea* have partial immunomodulate activity. Their molecular weights were around 13 kD, and neither of them contains histidine, cysteine or methionine. They are a kind of lectins that are linked to carbohydrates.

Natural Killer (NK) cells are yet another type of lethal lymphocyte. Like cytotoxic T cells, they contain granules filled with potent chemicals. They are called "natural" killers because they, unlike cytotoxic T cells, do not need to recognize a specific antigen before swinging into action. They target tumor cells and protect against a wide variety of infectious microbes. In several immunodeficiency diseases, including AIDS, natural killer cell function is abnormal. Natural killer cells may also contribute to immunoregulation by secreting high levels of influential lymphokines.

Both cytotoxic T cells and natural killer cells kill on contact. The killer binds to its target, aims its weapons, and then delivers a lethal burst of chemicals that produces holes in the target cell's membrane. Fluids seep in and leak out, and the cell bursts.

Until recently, immuno-anti-cancer therapy consisted of three forms: operations, chemotherapy, or radiation. In all these forms, however, resulting side effects were frequent and harmful. Thus, these three forms are not the best way for cancer patients, especially those people in the end stages of cancer. Overdoses of chemotherapy and radiation, for example, could actually prove harmful and shorten lives.

In recent years, however, a fourth anti-cancer immuno-therapy has become popular. This fourth way actually strengthens each patient's own natural anti-cancer immuno-power. This fourth way uses the body's own NK (natural killer) cells, which are the strongest and most effective immune cells in the body. There are almost 50,000 times stronger than Killer T- cell, This NK immuno-therapy would undoubtedly become more and more popular in the future.

NK cells constitute an important component of the innate immune system, providing surveillance against certain viruses, intracellular bacteria and transformed cells (Trinchieri G. Adv Immunol 1989; 47:187- 376.; French AR, Yokoyama WM. Curr Opin Immunol 2003; 15:45 - 51.; Smyth MJ et al., Nat Immunol 2001; 2:293 - 9.). NK cells exert cell-mediated cytotoxicity and stand as a bridge between innate and adaptive immune responses through the release of various cytokines (such as IFNg, GM-CSF and TNF-h) and chemokines (e.g. MIP-1 family and RANTES) (Biron CA. Curr Opin Immunol 1997; 9:24- 34.; Biron CA et al., Annu Rev Immunol 1999; 17:189- 220.). Unlike T cells, NK cell killing of virus-infected or malignant transformed cells do not need pre-sensitization and is independent of MHC restriction, thus NK cells are considered as promising candidates for adoptive transfer treatment of malignant tumors, especially those of the haematopoietic origin (Robertson MJ, Ritz J. Blood 1990; 76:2421 - 38.). Tumor cells that lose or express altered MHC class I antigen escape detection by cytotoxic CD8+ T cells, but they are likely susceptible to be eliminated by NK cells. However, malignant cells often have developed strategies that counteract immune surveillance of the hosts, including down-regulation of MHC class I molecules to avoid immune recognition, increased expression of Fas-L to kill responsive lymphocytes and production of suppressive cytokines such as TGF-h (Garcia-Lora A et al., J Cell Physiol 2003; 195:346-55.; Kim R et al., Cancer 2004; 100:2281 - 91.). Therefore, mobilizing NK cells is important to increase the capacity of the host to limit the development of malignant tumors while adaptive immunity is at the states of "anergy" or "tolerance".

Macrophages and neutrophils both can be regarded as heroes and villains on tumor development. These cells are capable of phagocytosis of and antibody-dependent cellular cytotoxicity (ADCC) towards tumor cells, and secretion of tumor-growth inhibitory cytokines (Marek Jakóbisiak et al., Immunology Letters December 15, 2003 pp: 103-122).

Recently, herbal therapies have increasingly been considered viable alternative treatments for malignancies (Eisenberg DM, et al., Jama 1998; 280(18):1569-1575.; Risberg T, et al., J Clin Oncol 1998; 16(1):6-12.). Of these therapies, medicinal mushrooms have a long history of use in folk medicine worldwide and in Asia *Ganoderma tsugae* (*G. tsugae*)*,* a basidiomycetes mushroom, is one of the most popular chemopreventive mushrooms. Many bioactive components have been identified from the different parts of this mushroom, including the fruiting body, mycelia, spores and culture media.

Two major categories of bioactive ingredients are polysaccharides and triterpenes. *G. lucidum* has polysaccharides which, through an immune-modulatory mechanism, have *in vitro* and *in vivo* anticancer effects (Wang SY, et al., Int J Cancer 1997; 70(6):699-705.). Some researchers have reported that triterpenes generally possess antioxidation (Zhu M, Chang Q, et al., Phytother Res 1999; 13(6):529-531.), hepatoprotection (Kim DH, et al., Biol Pharm Bull 1999; 22(2):162-164.) and anti-hypertension (Kabir Y, et al., J Nutr Sci Vitaminol (Tokyo) 1988; 34(4):433-438.) bioactivity. Recently, cytotoxic activity against tumor cells was reported from *Ganoderma spp.* One *Ganoderma tsugae* triterpene was found to induce cell apoptosis and cell cycle arrest in human hepatoma Hep3B cells, but the molecular mechanism was not investigated (Gan KH, et al., J Nat Prod 1998; 61(4):485-487).

Telomerase is a cellular reverse transcriptase that catalyzes the synthesis and extension of telomeric DNA (Greider CW, et al., Nature 1989; 337(6205):331-337.). This enzyme is specifically activated in most malignant tumors but is usually inactive in normal somatic cells, with the result that telomeres are progressively shortened during cell division in normal cells (Kim NW, et al., Science 1994; 266(5193):2011-2015.). Cells require a mechanism to maintain telomere stability to overcome replicative senescence, and telomerase activation may therefore be a rate-limiting or critical step in cellular immortalization and oncogenesis (Harley CB, et al., Curr Opin Genet Dev 1995; 5(2):249-255.), as more than 90% of human cancer cells in vivo show the presence of telomerase activity. As a ribonucleoprotein complex, telomerase in humans consists of two major subunits. These are the RNA template and the reverse transcriptase subunit, encoded by hTR and hTERT genes, respectively. Interestingly, lung cancer patients without telomerase activity survive for a significantly better prognosis than those with telomerase activity (Wu TC, et al., Lung Cancer 2003; 41(2):163-169.). This suggests that telomerase activity is an important prognostic factor in lung cancer patients.

### Summary of the Invention

The present invention is directed to an use of the fungal immunomodulatory protein for immunotherapy, wherein the protein having the amino acid sequence of SEQ ID No:1
MSDTALIFRLAWDVKKLSFDYTPNWGRGNPNNFIDTVTFPKVLTDKAYTYRV AVSGRNLGVKPSYAVESDGSQKVNFLEYNSGYGIADTNTIQVFVVDPDTNND FIIAQWN
or
SEQ ID No:3
SATSLTFQLAYLVKKIDFDYTPNWGRGTPSSYIDNLTFPKVLTDKKYSYRVVV NGSDLGVESNFAVTPSGGQTINFLQYNKGYGVADTKTIQVFVVIPDTGNSEEYI IAEWKKT.

### Brief Descriptions of the Drawings

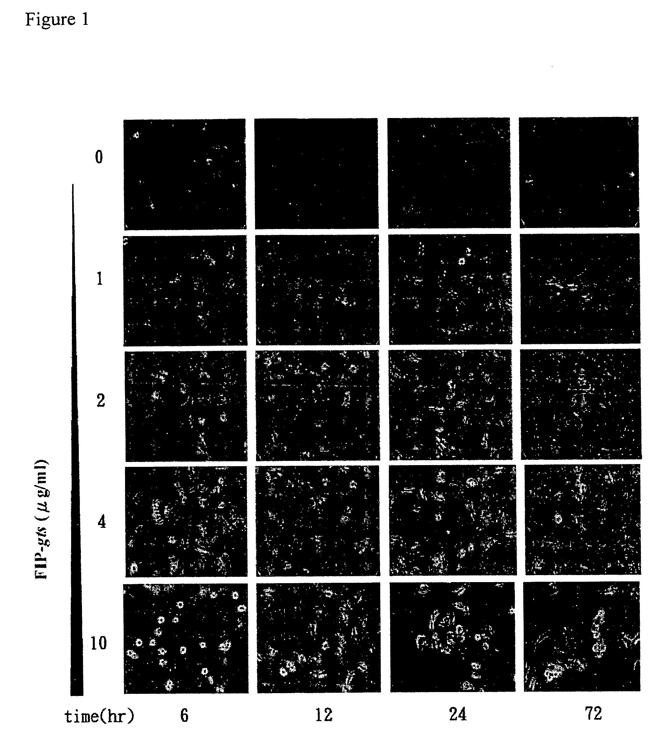

Figure 1 discloses the morphological change of A549 cells treated with FIP-*gts.* A549 cells were treated with different concentrations of FIP-*gts* (0, 1, 2, 4 and 10 µg/ml) at different durations (6, 12, 24 and 72 hrs).

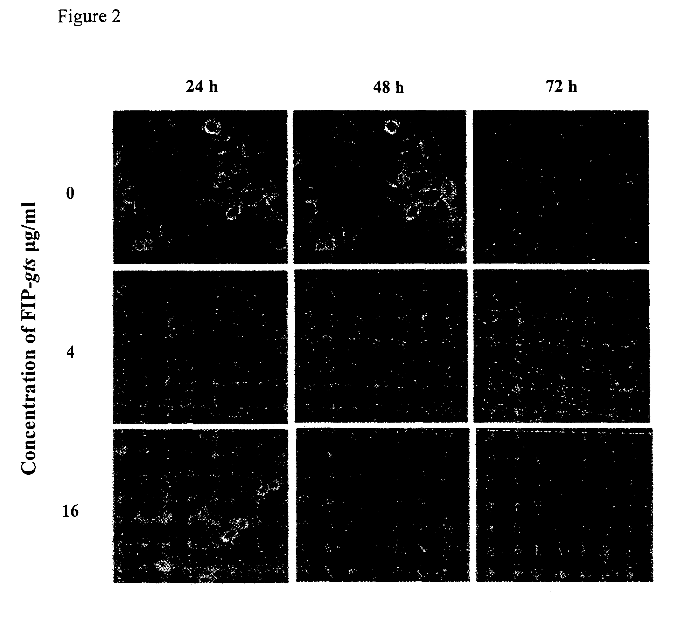

Figure 2 discloses the morphology changes of human melanoma cancer cell line A375 after they were treated with FIP-*gts.* Cells were treated with 0, 4 and 16 µg/ml FIP-*gts* for 0, 24 and 48 hours and photographed with a phase-contrast microscope (x100).

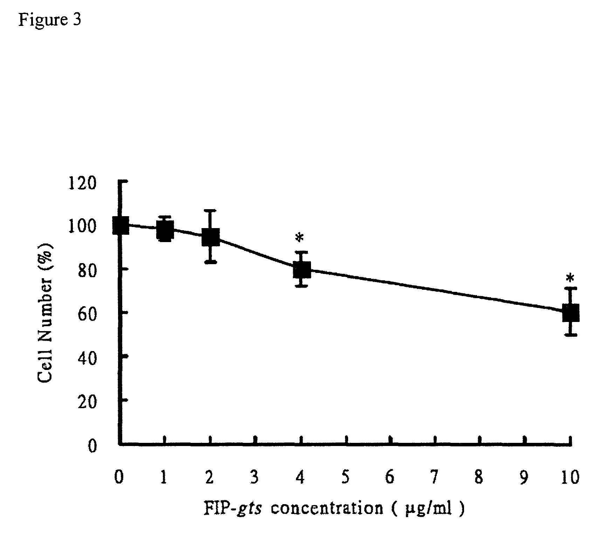

Figure 3 discloses the growth rate of A549 cells treated with FIP-*gts* at different times. A549 cells were treated with 0, 1, 2, 4 and 10 µg/ml FIP-*gts* and viable cell numbers were measured using trypan blue dye exclusion method at 48 hrs. The data shown here are mean ± standard deviation of triplicate experiments (significance calculated using student T test, * p<0.05).

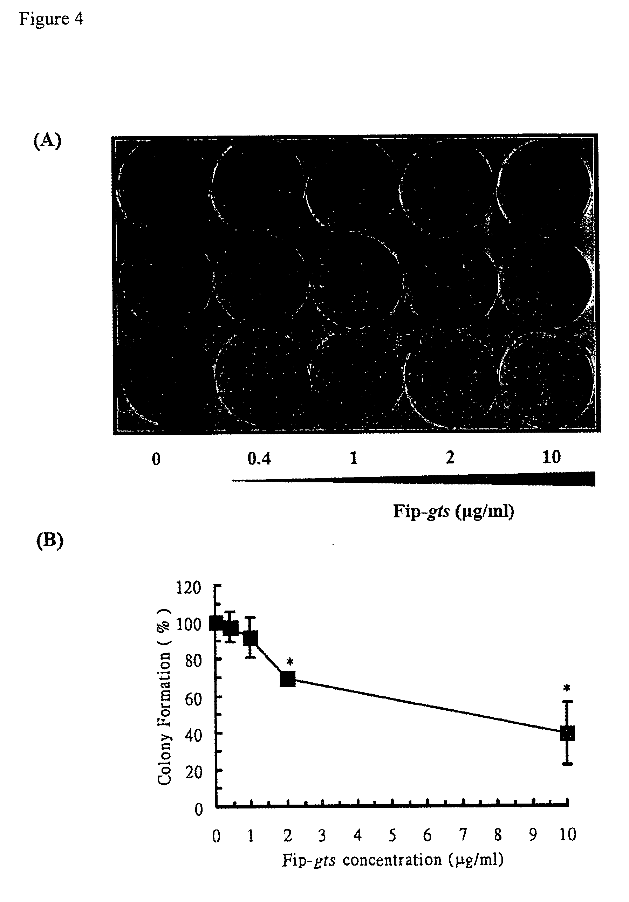

Figure 4 shows the effect of FIP-*gts* on the colony formation of A549 cells. (A) Anchorage-independent growth of A549 cells treated with 0, 0.4, 1 and 2 µg/ml FIP-*gts* was assessed by the colony formation assay. (B) The colony number was counted under a dissection microscope. The number of cells has to be greater than 50 cells per colony. The data shown here are mean ± standard deviation of triplicate experiments (significance calculated using student T test, * p<0.05).

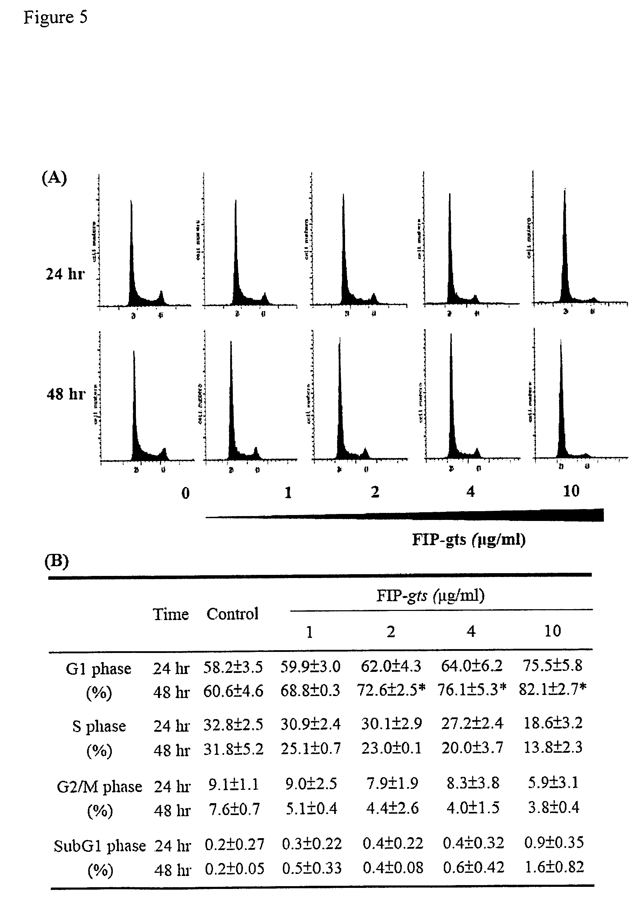

Figure 5 shows the stage of A549 cells in cell cycle treated with different doses and time courses of FIP-*gts.* Cells were resuspended in 10% DMEM medium at 2 X 10⁶ cells/ml. (A) Cells were detected by Flow cytometer and acquired by Cellquest. (B) Acquisition were analyzed and quantified by ModFit LT 3.0. The data shown here are mean ± standard deviation of triplicate experiments (significance calculated using student T test, * p<0.05).

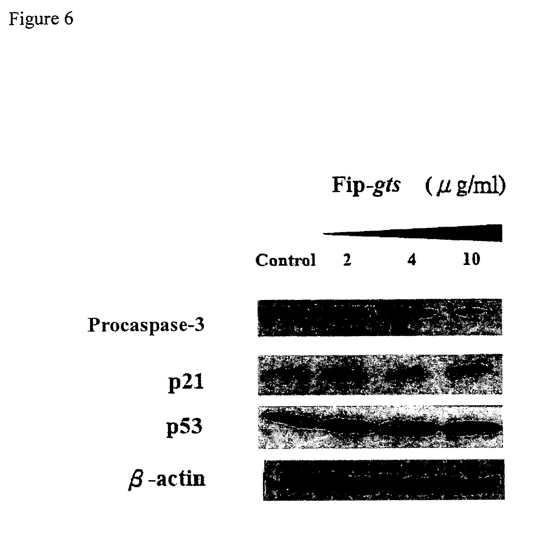

Figure 6 shows the expression of, p21 and procaspase-3 of A549 cells treated with 0, 2, 4 and 10 µg/ml FIP-*gts,* respectively. Cell lysates were collected at 48 hrs and expression were determined by Western blot analysis.

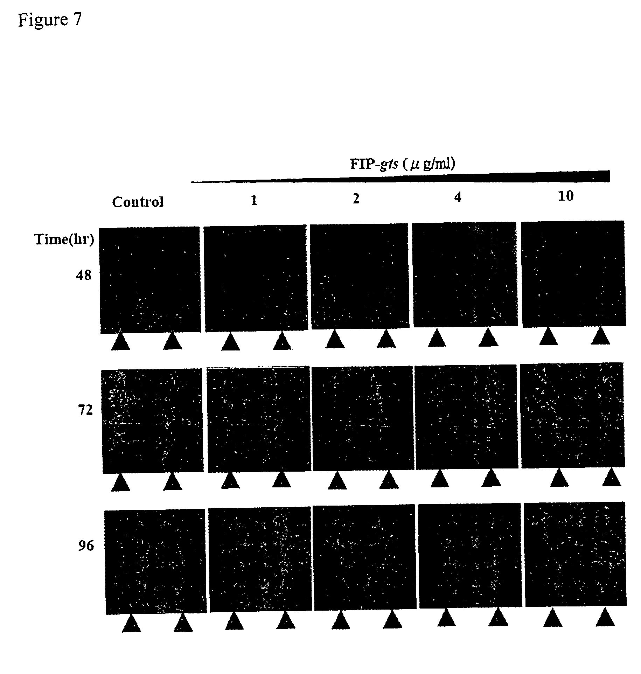

Figure 7 shows the migration of A549 cell treated with FIP-*gts* into the wound. Wounds were made by scarifying confluent A549 cells by a pipette tip (*arrowheads* show the size of the initial wound). After incubation for 72 h or 96 h cells were fixed and stained by Geimsa stain.

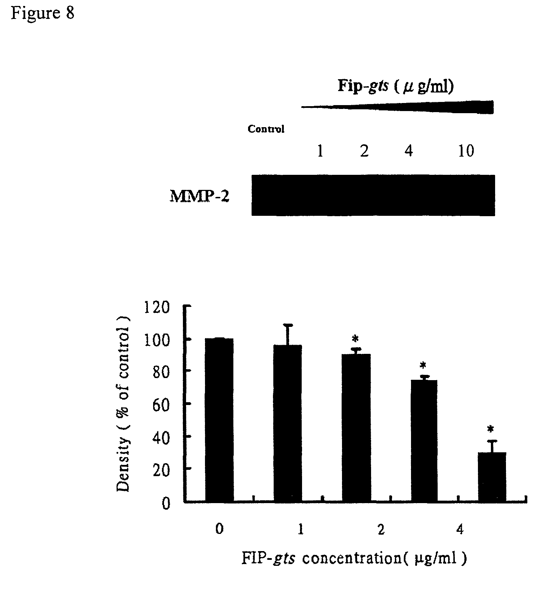

Figure 8 shows the activity of MMP-2 treated with FIP-*gts.* (A) A549 cells were treated with 0, 1, 2, 4 and 10 µg/ml FIP-*gts* for 24 hrs. The conditioned media were collected and MMP-2 activity was determined by gelatin zymography. (B) The activity of MMP-2 was quantified by densitomertic analysis. The densitomertic data shown here are mean ± standard deviation of triplicate experiments (significance calculated using student T test, * p<0.05).

### Detailed Description of the Invention

Unless defined otherwise, all technical and scientific terms used herein have the same meaning as commonly understood by one of ordinary skill in the art to which this invention belongs. Although any methods or materials similar or equivalent to those described herein can be used in the practice or testing of the present invention, the preferred methods and materials are described. For the purposes of the present invention, the following terms are defined below.

The present invention is provided an use of the fungal immunomodulatory protein for immunotherapy, wherein the protein having the amino acid sequence of SEQ ID No: 1
MSDTALIFRLAWDVKKLSFDYTPNWGRGNPNNFIDTVTFPKVLTDKAYTYRV AVSGRNLGVKPSYAVESDGSQKVNFLEYNSGYGIADTNTIQVFVVDPDTNND FIIAQWN
or
SEQ ID No:3
SATSLTFQLAYLVKKIDFDYTPNWGRGTPSSYIDNLTFPKVLTDKKYSYRVVV NGSDLGVESNFAVTPSGGQTINFLQYNKGYGVADTKTIQVFVVIPDTGNSEEYI IAEWKKT.

The use of the present invention could be obtained from *Ganoderma* species, *Flammulina velutipes* or a recombinant microorganism (such as recombinant *Escherichia coli* or Yeast). The preferred embodiment of *Ganoderma* species is *Ganoderma tsugae.*

The use of the present invention could be applied as adjuvant for alleviating the pain or side effects of a patient suffering cancer.

The use of the present invention for immunotherapy is directed to stimulate or activate immunological function.

Accordingly, the immunological function of the present invention is made by the mechanism selecting from the group consist of activation of natural killer cells, activation of macrophages and multiplication of serum antibodies. The preferred embodiment of the function of the present invention is made by activation of natural killer cells and inhibition of telomerase.

The fungal immunomodulatory protein of the present invention can be designed as the following dosage forms consisting of oral solution, oral sachet, oral tablet or oral pellet, which is administered orally.

It has been found that FIP-*gts* of the present invention, of which cDNA sequence is identical to LZ-8 (SEQ ID NO: 1), exhibited anti-cancer effect. It was also disclosed that cancer cells treated with FIP-*gts* showed reduced viability, demonstrating the utility of FIP-*gts* as an anticancer agent.

It has been found that cancer cells treated with FIP-*gts* of the present invention show a decrease of MMP-2 expression. MMP-2 is an important enzyme involved in the tumor cell metastasis. Suppression of MMP-2 is a sign of FIP-*gts* suppressing the tumor cell metastasis.

The term "immunotherapy" is not limited but to stimulate or activate immunological function (such as activate natural killer cells and macrophages or increase production of cytokines, serum IgG or IgM antibody).

Cancers the fungal immunomodulatory protein of the invention could treat are selected from the group consisting of lung cancer, bone cancer, breast cancer, hepatocellular carcinomas, non-small lung cell cancer, ovarian cancer and gastrointestinal cancer.

In another embodiment, the FIP-*gts* according to the invention could be fused to an antitumor agent or a detectable label. This allows the FIP-*gts* to target the agent or detectable label to the tumor cells and hence allows damage/destruction or detection of the tumor. Thus, the FIP-*gts* is suitable for use in a method of treatment of the human or animal body by chemotherapy or surgery (e.g. radioimmunoguided surgery, RIGS), or in a method of diagnosis practiced on the human or animal body. In particular, the FIP-*gts* is suitable for use in treatment by surgery or therapy of a tumor, or in diagnosis of a tumor.

The antitumor agent linked to the FIP-*gts* may be any agent that destroys or damages a tumor to which the FIP-*gts* has bound or in the environment of the cell to which the FIP-*gts* has bound. For example, the antitumor agent may be a toxic agent such as a chemotherapeutic agent or a radioisotope, an enzyme that activates a prodrug or a cytokine.

Suitable chemotherapeutic agents are known to those skilled in the art and include anthracyclines (e.g. daunomycin and doxorubicin), methotrexate, vindesine, neocarzinostatin, cis-platinum, chlorambucil, cytosine arabinoside, 5-fluorouridine, melphalan, ricin and calicheamicin.

Suitable radioisotopes for use as anti-virus agents are also known to those skilled in the art.

The antitumor agent that is attached to the FIP-*gts* may also be an enzyme that activates a prodrug. This allows activation of an inactive prodrug to its active, cytotoxic form at the directed site. In clinical practice, the FIP-*gts*-enzyme conjugate can be administered to the patient and allowed to localize in the region of the tumor to be treated. The prodrug is then administered to the patient so that conversion to the cytotoxic drug is localized in the region of the tumor cells to be treated under the influence of the localized enzyme.

Accordingly, the present invention also provides a method for use in immunotherapy in a patient in need of such treatment, comprising administering to said patient an effective amount of the polypeptide variant or fragment of the present invention.

The detectable label attached to the FIP-*gts* may be an imaging agent for site imaging such as a short-lived radioisotope, for example ¹¹¹In, ¹²⁵I or ⁹⁹mTc.

The FIP-*gts* according to the invention containing a detectable label is useful for RIGS in addition to being useful for diagnosis. RIGS comprises administering a labeled protein to a patient and thereafter surgically removing any tissue to which the protein binds. Thus, the labeled FIP-*gts* guides the surgeon towards tissue.

At present, telomerase inhibition research focuses on (1) direct targeting of core telomerase components (Kondo S, et al., Oncogene 1998; 16(25):3323-3330.; Hahn WC, et al., Nat Med 1999; 5(10):1164-1170.); (2) telomere targeting (Rezler EM, et al., Curr Opin Pharmacol 2002; 2(4):415-423.; Zhang RG, et al., Cell Res 2002; 12(1):55-62.); (3) natural compounds and small molecules as telomerase inhibitors (Lyu SY, et al., Arch Pharm Res 2002; 25(1):93-101.; Naasani I, et al., Biochem Biophys Res Commun 1998; 249(2):391-396.) and (4) interference with regulatory mechanisms of telomerase (Kawagoe J, et al., J Biol Chem 2003;278(44):43363-43372.).

Those skilled in the art may reasonably expect that the subjects or patients, to which these methods are directed, can be any vertebrate animals, most preferred patients are humans having cancer or at risk for cancer. Nonetheless, the utility of the methods toward any vertebrate can be determined without undue experimentation by administering the composition comprising FIP-*gts* to a cultured cancer cell specific to the vertebrate in question and performing a simple cellular invasion assay, heal wounded assay described in the example.

The composition comprising FIP-*gts* may be administered to a vertebrate by any suitable route known in the art including, for example, intravenous, subcutaneous, intratumoral, intramuscular, transdermal, intrathecal, or intracerebral. Administration can be either rapid as by injection, or over a period of time as by slow infusion or administration of a slow release formulation.

It is contemplated that the compositions comprising FIP-*gts* are usually employed in the form of pharmaceutical preparations. Such preparations are made in a manner well known in the pharmaceutical art. One preferred preparation utilizes a vehicle of physiological saline solution; it is contemplated that other pharmaceutically acceptable carriers such as physiological concentrations of other non-toxic salts or compounds, 5% aqueous glucose solution, sterile water or the like may also be used. It may also be desirable that a suitable buffer be present in the composition. Such solutions can, if desired, be lyophilized and stored in a sterile ampoule ready for reconstitution by the addition of sterile water for ready injection. The primary solvent can be aqueous or alternatively non-aqueous.

The carrier can also contain other pharmaceutically-acceptable excipients for modifying or maintaining the pH, osmolarity, viscosity, clarity, color, sterility, stability, rate of dissolution, or odor of the formulation. Similarly, the carrier may contain still other pharmaceutically-acceptable excipients for modifying or maintaining release or absorption or penetration across the blood-brain barrier. Such excipients are those substances usually and customarily employed to formulate dosages for parenteral administration in either unit dosage or multi-dose form or for direct infusion by continuous or periodic infusion.

It is also contemplated that certain formulations comprising the compositions that comprises FIP-*gts* are to be administered orally. Such formulations are preferably formulated with suitable carriers, excipients, lubricants, emulsifying agents, suspending agents, sweetening agents, flavor agents, preserving agents and pressed as tablet or encapsulated as solid capsule or soft capsule. Or it is contemplated that such formulations are designed as following dosage forms, either oral solution, or oral sachet, or oral pellet. Or apart from being administered orally, it is contemplated that such formulations are designed as enema, or suppository, or implant, or patch, or cream, or ointment dosage forms. Some examples of suitable carriers, excipients, and diluents include lactose, dextrose, sucrose, sorbitol, mannitol, starches, gum acacia, calcium phosphate, alginates, calcium silicate, microcrystalline cellulose, polyvinylpyrrolidone, cellulose, gelatin, syrup, methyl cellulose, methyl- and propylhydroxybenzoates, talc, magnesium, stearate, water, mineral oil, and the like. The formulations can additionally include lubricating agents, wetting agents, emulsifying and suspending agents, preserving agents, sweetening agents or flavoring agents. The compositions may be formulated so as to provide rapid, sustained, or delayed release of the active ingredients after administration to the patient by employing procedures well known in the art. The formulations can also contain substances that diminish proteolytic, nucleic acid and other degradation and/or substances that promote absorption such as, for example, surface active agents. Compositions may be complexed with polyethylene glycol (i.e., PEGylated), albumin or the like to help promote stability in the bloodstream.

The compositions comprising FIP-*gts* are administered to vertebrates in an amount effective to decrease the growth or metastasis of a tumor within the vertebrate. The specific dose is calculated according to the approximate body weight or body surface area of the patient or the volume of body space to be occupied. The dose will also be calculated dependent upon the particular route of administration selected. Further refinement of the calculations necessary to determine the appropriate dosage for treatment is routinely made by those of ordinary skill in the art. Such calculations can be made without undue experimentation by one skilled in the art in light of the activity disclosed herein, i.e., the gelatin-zymography assay. It will be understood that the amount of the composition actually administered will be determined by a practitioner, in the light of the relevant circumstances including the condition or conditions to be treated, the choice of composition to be administered, the age, weight, and response of the individual patient, the severity of the patient's symptoms, and the chosen route of administration. Dose administration can be repeated depending upon the pharmacokinetic parameters of the dosage formulation and the route of administration used.

The compositions comprising FIP-*gts* are fed to BALB/c mice to examine the effect on natural killer activity, macrophage activity, cytokines and serum antibody production. As compared with various dosage groups, it demonstrates that FIP-*gts* promotes the activities of natural killer cells, macrophage activity, cytokines and serum antibody production.

The following examples are included in the specification to help illustrate the invention and are not meant to limit the scope of the invention.

### Examples

### Example 1 Changes of cell morphology

Human pulmonary epithelial cancer cell A549 was a highly vicious pulmonary cancer cell line with high migratory capability. A549 cells as the model system was applied to examine the effect of treating or preventing cancer cells with FIP-*gts.*

First, the change of cell morphology after cells were treated with FIP-*gts* under the microscope. A549 Cells were treated with 0, 1, 2, 4 and 10 µg/ml FIP-*gts,* respectively, and photographed at different time interval (Figure 1).

It was found that after cells were treated with 2, 4 and 10 µg/ml FIP-*gts,* the morphology of cells clearly changed at 6 hours. Cells transformed from adhering with little tentacles to round and loosly-attached cells. Some cells showed signs of moving when the culture dish was shaked. After treated with FIP-gts for 72 hours, A549 cells not treated or treated wtih low concentrations of FIP-*gts* expanded and covered the whole culture dish, whereas cells treated with high concentrations of FIP-*gts* showed round shape and left many spaces uncovered (Figure 2). Normal lung cell line BEAS-2B cells treated with 0, 2, 4 and 10 µg/ml FIP-*gts* did not show morphology changes. And cells treated or not treated with FIP-*gts* exhibited similar growth rate in 24 hours, filling the culture dish almost concurrently. The control, BEAS 2B cell, were not affected by FIP-*gts* and filled the whole culture dish in 24 hours.

Using human melanoma cancer cell line A375, it has been found that A375 cells treated with FIP-*gts* seemed to lose cell-to-cell adhesion, as cells were no longer closely attached to each other but widely dispersed (Figure 2). A375 cells were treated with FIP-*gts* 0, 4 and 16 µg/ml and observed after 24, 48 and 72 hours. It has been found that more cells changed to round shape when higher concentrations of FIP-*gts* were used (Figure 2). It was shown that 16 µg/ml of FIP-*gts* could significantly inhibit cell adhesion and cell growth. Given the above, it proved that FIP-*gts* changed cell migratory and adhesion capability by rearranging the cell frame.

### Example 2 Cell viability assay

Trypan blue was used to examine cell viability. The same concentration of FIP-*gts* in Example 1 was used to treat A549 cells. After treating cells with FIP-*gts* for 48 hours, trypan blue was added. Live cells could repel the trypan blue; therefore, the number of viable cells was measured by the number of cells not labeled by trypan blue.

2×10⁵ human lung epidermoid carcinoma cell line H1355 and A549 cells were inoculated to 6 cm culture dishes. H1355 cell line was a common cellular model for studying metastasis. Cells were grown at 37°C for 16 hours. Medium was removed and FIP-*gts* at the concentrations of 0, 2, 4 and 10 µg/ml were treated.

Cells were collected at 48 hours after FIP-*gts* treatment. Cells were collected by removing the old culture medium into 15 ml centrifuge tube. Cells were washed with 1 ×PBS twice. Cells were resuspended in 0.5 ml TE buffer after centrifugation at room temperature for 1 min. The solution was neutralized by adding the original culture medium. Cells were transferred to a 15 ml centrifugation tube, and centrifuged at 800 rpm for 5 min. Supernatant was then discarded and cells were dispersed with 0.5 ml 1 ×PBS. 20 µl of cell culture was added with 5 µl Trypan blue solution. Cell numbers were counted with cell counters.

The survival rate of cells treated with 0 µg/ml FIP-*gts* for 48 hours was considered 100 %, and the survival rate of cells treated with 1, 2, 4 and 10 µg/ml FIP-*gts* for 48 hours were 98.2 %, 94.8 %, 80.0 % and 60.3 %, respectively (Figure 3). The result was consistent with the observation of the MTS assay (described below). These two experiments demonstrated that FIP-*gts* had cytotoxicity to A549 cells, and might suppress cell growth or cause the decrease of cells survival rate.

### Example 3 Counting cell numbers --- colony formation

The purpose of the present experiment was to examine the cytotoxicity of FIP-*gts* by colony formation assay. A549 or A375 cells were treated with 0, 0.4, 2 and 10 µg/ml FIP-*gts* for 24 hours. And then 400 cells/60 mm dishes were grown for 12 days.

2×10⁵ A549 or A375 cells were inoculated into 60 mm culture dish. Cells were grown at 37°C for 16 hr. FIP-*gts* at the concentrations of 0.4, 1, 2 and 10 µg/ml were treated to A549 cells (Figure 4). After growing for 24 hours and washing with 1×PBS twice, cells were subcultured to new plates. 1ml TE buffer was added and culture was let aside at 37°C for 1 min to distach the cell. Cell numbers were counted and series cell dilution was performed with the original culture medium. 400 cells/plate cells were inoculated into 6 cm culture plates. Cells were grown in 37°C incubators for 12 days. Cells were washed with 1 ×PBS twice and 0°C, 95% ethanol 2 ml was added into each plate. Culture was let aside at room temperature for 20 min. Ethanol was then discarded and 2 ml/plate 10% Geimsa stain (Geimsa stain diluted with ddH₂O) was added to each plate. Reaction was let aside at room temperature for 30 min. Dye was recollected and the remaining dye was gently washed out with tap water. Colony numbers were measured after dry.

The survival rate of A549 cells treated with 0 µg/ml FIP-*gts* was considered 100 %, and the survival rate of cells treated with 0.4, 1, 2 and 10 µg/ml FIP-*gts* were 97.3 %, 91.5 %, 69.6 % and 39.0 %, respectively (Figure 4A, 4B). All experiment groups except cells treated with 1 µg/ml FIP-*gts* showed significant decrease of survival rate (analyzed by student T test, p<0.05). It proved that FIP-*gts* showed cytotoxicity to A549 cells and suppressed colony formation.

### Example 4 Flow cytometry

It has been found that cells treated with FIP-*gts* showed lower survival rate. The effect might be the result of growth suppression or increased apoptosis. Traditionally anti-cancer drug suppressed cancer by modulating cell cycle, particularly arrested the cell at G1 phase. Therefore it examined whether treated A549 cells with FIP-*gts* modulate cell cycle, and if normal cell lines and cancer cell lines were differently affected by FIP-*gts.*

Cells were distributed into 60 mm culture dish, 5×10⁵ cells/dish with 5 ml culture medium, grown at 37°C for 16 hours. Old culture medium was discarded and washed twice with 1×PBS. Cells were treated with different concentrations of FIP-*gts* (0, 2, 4 and 10 µg/ml) at different time interval (24 hours and 48 hours). Cells were collected by the following procedures:
a. Old culture medium was moved to 15 ml centrifuge tube.
b. Cells were washed with cold 1 ×PBS twice.
c. Cells were distached by treating cells with 1 ×Trypsin-EDTA.
d. Old culture medium was added to stop the reaction, medium was moved to 15 microcentrifuge tube.
e. Cells were centrifuged at 800 rpm for 5 min. Supernatant discarded, pellet washed with 1 ×PBS twice.
f. 1 ml 70 % cold ethanol was slowly added to the culture. Cells were left at 4°C overnight to stabilize.
g. Culture was centrifuged at 800 rpm for 5min. Supernatant discarded.
h. Culture was washed with ice cold 1×PBS twice. Supernatant was discarded and let dry as mich as possible.
i. 1 ml propidium iodide (PI) mixture to each tube was added to each tube (avoid light) :
   1XPBS 550 µl
   5% Triton X-100 200 µl
   250 µg/ml propidium iodide 200 µl
   0.5 mg / ml RNase A 50 µl
j. Sample was let aside at room temperature for 30 min.
k. Culture was filtered with 40 µm nylon mesh to avoid oversized cell clusters or debris stuck the entrance hole of flow cytometer. Single cell suspension in flow cytometer tube was collected.

To measure the DNA in cells, the experiment used Fluorescence-Activated Cell Sorter (FACS) system to sort cells and analyzed with FACSCalibur (BECTON DICKINSON). The absorbance of red fluorescence at 617 nm determined the DNA content of cells labeled with PI. The measurement was analyzed by program CELL Quest. The statistics were computed and cell numbers at different stages of cell cycle were displayed using program Mod Fit 3.0.

Using flow cytometer, it has been found that cells treated with FIP-*gts* showed a profound arrest at G1 phase. At most more than 30 % cells were found to be at G1 phase. The G1 phase arrested decreased the proportion of cells at S phase. In other words, cell growth was suppressed by FIP-*gts.* Fewer cells were found to be at SubG 1 phase. The highest proportion of cells at subG1 phase (1.6 %) were found at the second day after cells were treated wtih high concentraitions of FIP-*gts.* The phenomenon suggested that FIP-*gts* lowered the cell curvival rate by causing G1 phase arrest and minor apoptosis.

The results showed that the higher concentrations of FIP-*gts* treated, the more cells arrested at the G1 phase. A549 cells treated with 0, 1, 2, 4 and 10 µg/ml FIP-*gts* for 24 hours showed a proportion of 58.2 %, 59.1 %, 62.0 %, 64.0 % and 75.5 % cells at the G1 phase, respectively. The increase of G1 phase also caused the decrease of cells at their S phase. A549 cells treated with the same concentrations of FIP-*gts* as above showed a proportion of 32.8 %, 30.9 %, 30.1 %, 27.2 % and 18.2 % at their S phase (Figures 5A and 5B), respectively. The cells arrested at G 1 phase also increased as the time of FIP-*gts* treatment increased. A549 cells treated with the same concentrations of FIP-*gts* as described above for 48 hours showed a proportion of 60.2 %, 68.8 %, 72.6 %, 76.1 % and 82.1 % at their G 1 phase, respectively, and the same cells showed an even lower proportion of cells at their S phase, respectively 31.8 %, 25.1 %, 23.0 %, 20.0 % and 13.8 %. Thus the experiment demonstrated that FIP-*gts* caused A549 cells to arrest at G 1 phase (Figures 5A and 5B).

It has been found that few cells were at their SubG 1 phase when A549 cells were treated with 10 µg/ml 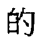 FIP-*gts.* Moreover, fewer cells went through apoptosis when they were treated with FIP-*gts.* 0.9% and 1.6% of A549 cells treated with FIP-*gts* 10 µg/ml for 24 hours and 48 hours, respectively, were at their SubG 1 phase (Figure 5B).

### Example 5 Western blot

External signals, such as UV, cisplatin could activate and stabilize p53 protein. p53 further activated other downstream genes including p21. p21 was the major checkpoint protein of the G1 phase in cell cycle (Zhong, X. et al, 2004. Int. J. Cancer). Using western blot, it has been found that the expression of p53 protein was induced after treating cells with FIP-*gts* for 48 hours. Gene p21 was also induced, demonstrating that FIP-*gts* caused cells to arrest at G1 phase by activating p21.

There were three routes of apoptosis: through ER, death receptor or mitochodria. All three pathways resulted in the cleavage of procaspase 3 (32 kD) into the active caspase 3 (17 kD). Caspase 3 was the final executor of the caspase series. It led to cell apoptosis, DNA breakage, nucleus condense and the formation of inclusions (Di Pietro, R., et al. (2004) Int J Immunopathol Pharmacol 17 (2)181-190). It has been found that when cells were treated with high concentrations of FIP-*gts,* there was a slight decrease of procaspase-3, the result of cleavage of procaspase-3 into active caspase-3.

From the results of the flow cytometer it has been found that FIP-*gts* caused cells to arrest at G1 phase and initiate minor apoptosis at high concentrations. Therefore the change of protein expression was studied when cells were treated with FIP-*gts*.

### a. sample preparation

A549 5×10⁵ cells/plate were inoculated to 60 mm culture dish. Cells were grown at 37 °C for 16 hours. Cells were treated with 0, 2, 4 and 10 µg/ml FIP-*gts* and grown at 37 °C incubator for 48 hours. Cells were first washed twice with PBS. Supernatant was discarded and 100 µl cell buffer (10 mM EDTA, 10 mM EGTA, 5 mM NaF, 10 % glycerol, 1 mM DTT, 400 mM KCl, 0.4 % Triton X-100, 20 mM sodium β-glycerophosphate, 0.1 mM Na₃VO₄, 1 mM PMSF/DMSO, 3 µg/ml aprotinin, 2 µg/ml pepstatin A, 2 µg/ml Leupeptin, 1X phosphatase inhibitor cocktail I (Sigma, P2850) were added ; 1X phosphatase inhibitor cocktail II (Sigma, P5726)) was also added to dissolve cells. The reaction was left on ice and cells were homogenized using ultrasonice homogenizer at 4 °C two times with an interval more than 10 minutes. Cells were centrifuged again at 12000 rpm, 4°C for 20 min. Supernatant was carefully moved to another sterilized 1.5 mlmicron and the protein content was quantified. The whole process of treating FIP-*gts,* centrifuging cells and adding 2X SDS sample buffer (200 mM Tris pH6.8, 8 % SDS, 40 % Glycerol, 2.86 M 2-mercaptoethenol and appropiate amount of bromophenol blue), to heat at 95°C, must be finished in 2 hours.

### b. protein quantification

Bio-Rad solution was applied to quantify protein concentration. First Bio-Rad reagent was diluted with dd H₂O 4:1, this was the Bio-Rad protein detection reagent. The sample, the supernatant of the cell culture 2 µl and the diluted Bio-Rad protein detection reagent 498 µl was mixed. Sample was reacted in 1.5 ml micron at 37°C for 20 min. The absorbance peak was measured by spectrophotometer at 595 nm and compared with the absorbance peack of standard sample bovine serum albumin (BSA) to get the protein quantity (µg/µl). The standard BSA quantity was measured by adding 2 µg, 4 µg, 6 µg, 8 µg and 10 µg BSA into diluted Bio-Rad protein detection reagent 498 µl, 496 µl, 494 µl, 492 µl and 490 µl, respectively. Sample was reacted in 1.5 ml micron at 37 °C for 20 min. The sample absorbance peak at 595 nm was also measured. The BSA measurement was measured to obtain the standard ccorrelation of protein quantity with peak absorbance. The protein quantity of the sample protein thus could be determined by putting in the peak absorbacnce of the sample.

### c. SDS-PAGE

**Table 1 The running gel was prepared as follows:**

| | 15 % | 12.5 % | 10 % |
|---|---|---|---|
| **dd H₂O** | 6.3 ml | 7.6 ml | 8.8 ml |
| **1.5M Tris pH 8.8** | 5 ml | 5 ml | 5 ml |
| **(38.67:1.33)Acrymide:Bis** | 7.5 ml | 6.2 ml | 5 ml |
| **10% SDS** | 0.2 ml | 0.2 ml | 0.2 ml |
| **APS(10 mg/ml)** | 1 ml | 1 ml | 1 ml |
| **TEMED** | 10 µl | 10 µl | 10 µl |
| Total Volume | 20 ml | 20 ml | 20 ml |

**Table 2 The 3% stacking gel was prepared as follows:**

| | | | |
|---|---|---|---|
| **dd H₂O** | 3.54 ml | | |
| **0.5M Tris pH 8.8** | 1.5 ml | | |
| **(38.67:1.33)Acrymide:Bis** | 0.45 ml | | |
| **10% SDS** | 0.06 ml | | |
| **APS(10 mg/ml)** | 0.3 ml | | |
| **TEMED** | 15 µl | | |
| **Total Volume** | 6ml | | |

The sample was run on SDS-PAGE, and Hybond-P membrane (Pharmacia) was prepared 20 minutes before electrophoresis finishes. The membrane was wet with methanol for 15 second, washed with ddH₂O for 10 min, and then the membrane was transferred to the transfer buffer (20 % methanol, 192 mM Glycine, 25 mM Tris-HCl, pH 9.2) for at least 10 min. The gel was carefully taken off after electrophoresis and transferred to Hoefer Semiphor following standard protocol. The transferred-membrane was blocked in shaking TTBS buffer (50 mM Tris, 0.2 % Tween 20, 150 mM NaCl, pH 7.5) with 5% nonfat milk powder for 1 hour.

### d. Antibody detection

Specific primary antibody was added to block Hybond-P membrane. 1 X TTBS buffer with 3 % BSA was added to dilute the following primary polyclonal antibody: anti-caspase-3 (1 : 500, Cayman), anti-COX-2(1 : 1000, Cayman #160106). 1X TTBS buffer with 5% nonfat milk powder was used to dilute the following primary antibody: anti-BAX (1 : 8000, R&D), p53 (1 : 500, DAKO), p21 (1 : 500, Zymed). Sample was shaken at 4 °C overnight (at least 16 hours). The membrane was taken out the other day. The membrane was washed with 100 ml 1X TTBS buffer with 3 % nonfat milk powder twice, 10 min each time. Anti-rabbit IgG-HRP (1 : 5000, Cell Signaling #7074) or anti-mouse IgG-HRP secondary antibody (1 : 10000, Chemicon AP124P) was diluted with 1X TTBS buffer with 3 % nonfat milk powder. Sample was shaken at room temperature for 1 hour. The washing procedure was repeated once. E.C.L. color development reagent (NEN, NEL105) was mixed 1 : 1 with Enhanced luminol reagent and Oxidizing reagent. The membrane was put face up into the container with color development reagent and let react for 5 min to develop the HRP color. The fluorescence was exposed on X-ray film for 3~5 min, and then developed and fixed the image.

The most important checkpoint of G1 phase was p21. It has been found that after treating with 0, 2, 4 and 10 µg/ml FIP-*gts* for 48 hours, p21 expression was significantly induced (Figure 6). It was also know that p21 was activated by p53, another well-known oncogene. The western blot result also showed that the expression of p53 was induced by FIP-*gts* (Figure 6). Therefore it proved that FIP-*gts* induced the expression of p53, increased the amount of p21 and caused G1 pause.

It has been found that procaspase-3 was decreased when cells were treated with 10 µg/ml FIP-*gts* (Figure 6). Thus procaspase-3 was activated into caspase-3 when cells were treated with FIP-*gts,* causing cells to undergo apoptosis. Moreover, the decrease of cells was not a result of enhanced apoptosis, but the result of suppression of proliferation.

### Example 6 Wound healing assay

Using wound healing assay, it has been found that FIP-*gts* could effectively suppress the mobility of breast cancer cells.

2×10⁵ A549 cells were gown in 24 well culture dish. Cells were grown until almost cover the culture plate and cells were treated with culture medium containing 0.5% FBS for 24 hours to suppress cell growth. The plate was scarified with blue tip and cells were washed with 1 ×PBS. Finally different concentrations of 1, 2, 4 and 10 µg/ml FIP-*gts* were added. Pictures were taken every 24 hour and cell migration was monitored.

Usually cancer cells obtained mobility before they performed metastasis. Wound healing assay was applied to examine whether treating cells with 0, 2, 4 and 10 µg/ml FIP-*gts* would increase the mobility of cells. It has been found that when cells were treated with FIP-*gts* for 48 hours, no significant mobility was observed. The invention identified cell migration that covered the line when cells were treated with 0, 1 and 2 µg/ml FIP-*gts* for 72 hours. Cells treated with 4 and 10 µg/ml FIP-*gts* almost showed no sign of migration. Compared cells treated or not treated with FIP-*gts* for 96 hours, cells not treated with FIP-*gts* showed substantial mobility and covered 1/3 of the scarified line, whereas cells treated with low concentrations of FIP-*gts* showed some migration, and cells treated with high concentrations showed no migration (Figure 7).

Using wound healing assay, it has been found that cell mobility were spuppressed when treated with FIP-*gts.* When the FIP-*gts* treated exceeded 4 and 10 µg/ml, A549 cells showed almost no mobility.

### Example 7 Gelatin Zymogragphy

During metastasis, metaloproteinase digested extracellular matrix, dissociated cells and extracellular matrix and provided cells mobility. It was known that metalloproteinases MMP-2 and MMP-9 were highly expressed in many vicious cancers (Johnsen, M., et al., Curr Opin Cell Biol, 1998. 10, 667-671). Therefore the expression of MMP-2 and MMP-9 and the metastasis of cancer cells were highly correlated (Curran, S. and Murray, G.I. Eur J Cancer, 2000. 36, 1621-1630., Liabakk, N.B., et al., Cancer Res, 1996. 56, 190-196.).

In order to avoid the interference of MMP-2 and MMP-9 in the serum, serum starvation on cell cultures and treated A549 cells with FIP-*gts* were applied to analyze the activity of MMP. To further improved the accuracy of gelatin zymography assay, Bio-Rad also been used to quantify protein concentration as a measure of cell density.

It has been found that cells treated with high concentrations of FIP-*gts* can suppress the expression of MMP-2. It also found that the effect of FIP-*gts* was dose-dependent.

A549 cells were grown 1 ×10⁵ cells/well in 24 well plate. Serum-free medium 200 µl/well were added the other day and cells were treated with 0, 2, 4 and 10 µg/ml FIP-*gts* for 24 hours. Medium was removed and cells were washed with 1X PBS. Cells were collected with CE buffer and proteins were quantified using Bio-Rad. 2 % gelatin was prepared by dissolving 2 g Gelatin /100 ml ddH₂O at 55°C.

**Table 3 8% SDS-PAGE gel was prepared with 0.1% Gelatin:**

| | 8% |
|---|---|
| **ddH₂O** | 3.0 ml |
| **1.5M Tris pH 8.8** | 2.0 ml |
| **(38.67:1.33) Acrymide:Bis** | 2.2 ml |
| **10 % SDS** | 0.08 ml |
| **APS (10 mg/ml)** | 0.4 ml |
| **2 % Gelatin** | 0.4 ml |
| **TEMED** | 10 µl |
| **Total Volume** | 8ml |

The gel was prepared as described in the western blot experiment. Gel was put in electrohporesis chamber with electrophoresis buffer. Culture media was loaded with 5X dye (0.1% SDS, 104 mM Tris-HCl pH 6.8, 50 % Glycerol (or 25 g sucrose), 0.125% bromophenol blue) into the gel and perform electrophoresis. Gel was then washed with washing buffer (40mM Tris-HCl pH 8.5, 0.2 M NaCl, 10 mM CaCl₂, 2.5% Triton X-100) at room temperature for 30 minutes twice, and reaction buffer (40mM Tris-HCl pH 8.5, 0.2M NaCl, 10 mM CaCl₂, 0.01% NaN₃) was added. Let react at 37°C incubator for 12 hours. The membrane was dyed with Coomassie blue (0.2 % Coomassie blue R-250, 50 % methanol, 10 % acetic acid) for 30 minutes. Gel was destained with 10% acetic acid and 20% methanol. Membrane was dried in 50 % ddH₂O, 50 % methanol and 0.33 % glycerol for 30 minutes. The membrane was then sealed in glass paper.

Because cell mobility is correlated to the expression of MMP, gelatin-zymograpghy was applied to analyze whether the activity of MMP-2 is altered by treating cells with FIP-*gts.* It has been found that the expression of MMP-2 significantly decreased when the amount of FIP-*gts* treated increases (student T test, *p<0.05). The expression of MMP when cells were not treated with FIP-*gts* was considered 100 %. It has been found that the expression of MMP treated with 1, 2, 4 and 10 µg/ml were 95.7 %, 90.3 %, 73.6 % and 29.8 %, respectively (Figure 8). Thus it concluded that FIP-*gts* modulates cell migratory through regulating the expression of MMP-2.

### Example 8 Reverse Transcriptase Polymerase Chain Reactions, RT-PCR

Since treated cells with high concentrations of FIP-*gts* shortly could caused the decrease of metalloproteinases expression, RT-PCR was applied to measure the mRNA expression of TIMP-1 (Tissue inhibitor of metalloproteinases), the inhibitor of metalloproteinases, after treating cells with FIP-*gts.* It has been found that the mRNA expression of TIMP-1 and PAI increased when cells were treated with FIP-*gts* 0, 2, 4 and 10 µg/ml for 24 hours. It also found that the mRNA expression of MMP-2 decreased but the expression of TIMP-2 was not affected. Thus it concluded that treated cells with FIP-*gts* caused the mRNA expression of MMP-2 to decrease, and activities of other MMPs such as MMP-9 would be suppressed by the increase expression of TIMP-1. Thus the cell metastasis was inhibited by treating cells with FIP-*gts.*

RT-PCR was performed by using Promega RT-PCR kit as follows:

1 µg total RNA was heated at 70°C. After 10 minutes, the heated RNA was cooled in ice bath. Then, 25mM MgCl₂ 4 µl, 5×MMLV buffer 4 µl, 10mM dNTP Mixture 2 µl, Recombinant RNasin Ribonuclease inhibitor 0.5 µl, MMLV Reverse transcriptase 1 µl, Oligo (dT)₁₅ Primer 1 µl and Nuclease-Free Water were added until the final volume was 20 µl.

**Table 4 Primers for performing RT-PCR:**

| Enzyme | Sequence 5'→3' | Position (bp) | Temp (°C) |
|---|---|---|---|
| MMP-2 | 5'-GGCCCTGTCACTCCTGAGAT-3' | 1337-1356 | 62°C |
| | 5'-GGCATCCAGGTTATCGGGGA-3' | 2026-2007 | |
| PAI-1 | 5'-GGATCCAGCCACTGGAAAGGCAACATG-3' | 1470-1490 | 55°C |
| | 5'-GGATCCGTGCCGGACCACAAAGAGGAA-3' | 1236-1216 | |
| TIMP-1 | 5'-TGGAGAGACACTGCCAACTTG-3' | 1700-1720 | 58°C |
| | 5'-AGGCTGTGCCTTCCTACAGA-3' | 2224-2204 | |

### Example 9 Increased cytokine expression.

Human peripheral blood mononuclear cells (PBMCs) were treated with 0, 1.25, 2.5, 5 and 10 µg/ml FIP-*gts.* After 48 hours the cytokine expression of human PBMCs was measured by ELISA. It has been found that the expression of cytokines IL-2, IFN-γ, TNF-α and IL-4 increased as the concentrations of FIP-*gts* treated increased (Table 5).

**Table 5. The increased cytokine expression of human PBMCs treated with FIP-gts.**

| | FIP-*gts* (µg/ml) | | | | |
|---|---|---|---|---|---|
| | 0 | 1.25 | 2.5 | 5 | 10 |
| IL-2 (pg/ml) | 116 | 316 | 272 | 425 | 1218 |
| IFN-γ (pg/ml) | 70 | 4135 | 4578 | 4378 | 4372 |
| TNF-α (pg/ml) | 89 | 1174 | 2076 | 3525 | 2219 |
| IL-4 (pg/ml) | 5 | 3 | 7 | 13 | 39 |

### Example 10 Comparing effects of FIP-gts on three different cell lines.

The effects of FIP-*gts* on 3 cancer cell lines: human prostate cancer cell line PC3, human breast cancer cell line MDA231 and human melanoma cancer cell line A375 were assessed (Table 6). The effects of FIP-*gts* were assessed by observing the morphology changes of cells treated with FIP-*gts,* following protocol described in Example 1; by measuring the inhibition of cell proliferation, following protocol described in Example 3; and by measuring the inhibition of colony formation, following the protocol described in Example 4.

**Table 6 Effects of FIP-gts on different cancer cell lines**

| **Cell line** | **Cell line origin** | **Morphology change** | **Inhibition of cell growth** | **Inhibition of colony growth** |
|---|---|---|---|---|
| PC3 | Human prostate cancer | n.d. | + | + |
| MDA231 | Human breast cancer | n.d. | + | + |
| A375 | Human melanoma cancer | + | + | + |

### Example 11

### Materials and methods

### Animal strain

BALB/c male mice, 4- to 5-week-old, were purchased from National Laboratory Animal Center in Taiwan.

### FIP-gts dosage (oral administration)

Lower dosage: 200 microgram /kg/day; higher dosage: 600 microgram/kg/day; positive dosage (a commercial Ling-Zhi powder purchased from Taiwan, Department of Health, Health Food Accredited No.A00003): 300 milligram/kg/day. Negative control: Distilled water.

### Feeding period, route and times

At first, the higher dosage of FIP-*gts* was formulated. Then, the lower dosage groups were diluted from the higher dosage group. From the first day for test, each group was fed with test materials by oral route once a day over 6 weeks.

### Assay

### 1. Natural Killer cells activity (non-specific immunomodulation function test)

After feeding FIP-*gts* experimental animals over six weeks, splenocytes was took out from the animals. Assay of natural killer cells activity by flowcytometry was made to compare various dosages groups with negative control group to check whether there was difference between the groups.

### 2. Macrophages activity (non-specific immunomodulation function test)

After feeding FIP-*gts* experimental animals over six weeks, macrophages in abdomen were took out from the animals. E. coli were labeled with fluorescence. Then, the macrophages were made to phagocytise the labeled E. coli. Assay of the macrophage activity by flowcytometry was made to compare various dosages groups with negative control group to check whether there was difference between the groups.

### 3. Production of serum antibody (non-specific immunomodulation function test)

During feeding FIP-*gts,* animals' blood was collected before FIP-*gts* treated and animal sacrifice. The concentrations of various immunoglobulins in serum were determined and various dosages groups with negative control group were compared to check whether there was difference between the groups.

### 4. Analysis of cytokine secretion (specific immunomodulation function test)

After ten weeks of FIP-*gts* administration and injecting ovalbumin (OVA) twice, animals were sacrificed. Splenocytes were stimulated by Concanavalin A (Con A), Lipopolysaccharide (LPS) or OVA. After 48~72 hours incubation, cytokine were measured by ELISA. When compared each dose group to negative group, p < 0.05 was an indication of significant difference.

### 5. Analysis of anti-OVA immunoglobulin production in serum (specific immunomodulation function test)

Before study of FIP-*gts,* first immunize, second boost, and before sacrifice, blood was collected from retro-orbital. Anti-OVA immunoglobulin production in serum was measured by ELISA. When compared each dose group to negative group, p<0.05 was an indication of significant difference.

### Result

### 1. Natural Killer Cells Activity (non-specific immunomodulation test)

After sacrificed, the splenocytes of mice were taken from to proceed with assay of natural killer cells activity. To compare with negative control group, each group showed no statistical significance under the ratio of Effector/ Target (E/T ratio) was 12.5. To compare with negative control group, higher dosage group and positive control group showed significant differences under E/T ratio was 25.0. To compare with negative control group, lower dosage group, higher dosage group and positive control group showed significant differences under E/T ratio was 50. It appeared that FIP-*gts* promoted the activity of natural killer cells (Table 7).

**Table 7**

| Group | animal number | E/T ratio | | |
|---|---|---|---|---|
| | | 12.5 | 25.0 | 50.0 |
| A | 10 | 17.5± 8.69 | 25.5± 8.16 | 26.9± 6.57 |
| B | 10 | 25.7± 8.59 | 34.9± 8.20* | 38.8± 6.80* |
| C | 10 | 23.9± 10.52 | 32.8± 7.92* | 38.1± 7.66* |
| D | 10 | 25.2± 9.85 | 33.9± 10.16* | 38.7± 9.22* |

| | | | | |
|---|---|---|---|---|
| This test is directed to the cytotoxicity assay of natural killer cells identified by flowcytometry. The symbol (*) indicates statistical significance, as compared with negative control group. E means effector cell. T means target cell. A means negative control group. B means lower dosage group. C means higher dosage group. D means positive control group. | | | | |

### 2. Macrophage Activity (non-specific immunomodulation test)

After sacrificed, the macrophages in abdomen of mice were collected. FITC-E. coli were added to make phagocytosis by the macrophages. Then, the activity of the macrophages was analyzed by flowcytometry. To compare with negative control group, the lower dosage group and higher dosage group showed statistical significance under Multiplicity of infection (MOI)=30. It appeared that FIP-*gts* promoted the activity of the macrophages in abdomen (Table 8).

**Table 8.**

| Group | animal number | MOI=30 | MOI=50 |
|---|---|---|---|
| A | 10 | 42.17± 8.89 | 46.33± 12.57 |
| B | 10 | 54.85± 8.73* | 52.30± 9.05 |
| C | 10 | 53.09± 15.73* | 49.74± 9.18 |
| D | 10 | 51.07± 8.43 | 46.11± 6.66 |

| | | | |
|---|---|---|---|
| This test is directed to macrophage phagocytosis identified by flowcytometry. The symbol (*) indicates statistical significance, as compared with negative control group. MOI means multiplicity of infection. A means negative control group. B means lower dosage group. C means higher dosage group. D means positive control group. | | | |

### 3. Production of Serum Antibody (non-specific immunomodulation test)

During feeding FIP-*gts,* animals' blood was collected before FIP-*gts* treated and animal sacrifice. The concentrations of various immunoglobulins in serum were determined and various dosages groups with negative control group were compared to check whether there was difference between the groups. The concentration of immunoglobulins in serum before treatment demonstrated that immunoglobulin G (IgG) of higher dosage group and positive control group showed statistical significance as compared with negative control group. Various dosages groups of IgM showed no statistical significance as compared with negative control group (Table 9).

**Table 9 Antibody production**

| Group | animal number | Ig G (µg/ml) | |
|---|---|---|---|
| | | before treatment | animal sacrifice |
| A | 10 | 431.06± 103.42 | 980.11± 163.89 |
| B | 10 | 504.22± 114.57 | 1324.55± 249.15* |
| | | | |

| Group | animal number | Ig M (µg/ml) | |
|---|---|---|---|
| | | before treatment | animal sacrifice |
| A | 10 | 356.87± 24.59 | 461.84± 103..5 |
| B | 10 | 333.17± 54.36 | 500.54± 46.09 |

| | | | |
|---|---|---|---|
| This test is directed to the condition of serum antibody production identified by ELISA. The symbol (*) indicates statistical significance (p<0.05), as compared with negative control group. A means negative control group. B means higher dosage group. | | | |

### 4. Cytokine secretion (specific immunomodulation test)

Splenocytes were stimulated by stimulant (Con A or LPS or OVA), after 48~72 hours incubation, cytokine was measured by ELISA. After Con A stimulation, interleukin-2 (IL-2) secretion in lower dose group of FIP-*gts* was significantly higher than that in negative control group. After OVA stimulation, interleukin-2 (IL-2) secretion in each dose and positive group were significantly higher than that in negative control group.

After Con A stimulation, tumor necrosis factor-alpha (TNF-α) secretion in each group and positive group were significantly higher than that in negative control group.
After OVA stimulation, interferon-gamma (IFN-γ) secretion in higher dose of FIP-*gts* and positive group was significantly higher than that in negative control group.

The results are shown in Table 10. The FIP-*gts* could promote cytokine secretion.

**Table 10 Spleen cytokine secretion**

| Group | Animal Number | Medium only | Con A | LPS | OVA |
|---|---|---|---|---|---|
| IL-2 (pg/ml) | | | | | |
| A | 10 | 10.71± 3.30 | 3224.48± 257.61 | 19.74± 6.64 | 16.68± 2.96 |
| B | 10 | 11.01± 2.91 | 3674.83± 800.84 | 21.52± 5.78 | 25.58± 7.18 |
| C | 10 | 12.28± 7.90 | 3783.18± 892.71 | 22.47± 4.76 | 26.90± 7.01* |
| D | 10 | 11.60± 5.26 | 3364.50± 494.03 | 21.05± 6.05 | 28.52± 14.01 * |

| TNF-α (pg/ml) | | | | | |
|---|---|---|---|---|---|
| A | 10 | 4.14± 5.21 | 14.26± 3.51 | 169.25± 104.31 | 4.93± 5.91 |
| B | 10 | 3.68± 4.67 | 18.29± 4.06* | 186.97± 84.15 | 6.00± 6.46 |
| C | 10 | 3.22± 5.92 | 19.94± 4.47* | 243.64± 146.35 | 6.15± 6.62 |
| D | 10 | 4.72± 6.02 | 18.77± 3.63* | 227.41± 122.00 | 5.90± 6.10 |

| IFN-γ (pg/ml) | | | | | |
|---|---|---|---|---|---|
| A | 10 | 8.29± 6.19 | 2297.73± 842.49 | 14.95± 9.64 | 4.16± 4.44 |
| B | 10 | 8.19± 5.19 | 2451.23± 879.89 | 16.78± 6.33 | 10.54± 4.35 |
| C | 10 | 8.45± 5.53 | 2404.08± 623.07 | 20.70± 16.67 | 18.67± 11.70* |
| D | 10 | 8.30± 5.37 | 2269.64± 328.37 | 15.12± 8.55 | 13.05± 10.52* |

| | | | | | |
|---|---|---|---|---|---|
| Spleen cytokine secretion were measured by ELISA *:°indication of statistical significance when compare to negative control A: Negative control B: Lower dose C: Higher dose D: Positive control | | | | | |

### 5. Anti-OVA immunoglobulin production in serum (specific immunomodulation)

Before study of FIP-*gts,* first immunize, second boost, and before sacrifice, blood was collected from retro-orbital. Anti-OVA immunoglobulin production in serum was measured by ELISA.

Before study, first immunize and second boost, there was no significant difference between each group in anti-OVA immunoglobulin G (IgG), anti-OVA immunoglobulin M (IgM) and anti-OVA immunoglobulin E (IgE).

Before animal sacrifice, anti-OVA IgG production in each dose of FIP-*gts* and positive group was significantly higher than that in negative control group. Anti-OVA IgM production in higher dose of FIP-*gts* and positive group was significantly higher than that in negative control group. There was no significant difference between each group in anti-OVA IgE.

The results are shown in Table 11. The FIP-*gts* could promote anti-OVA immunoglobulin production in sera.

**Table 11 Immunoglobulin production in serum (specific immunomodulation test)**

| Group | Animal Number | Before study | OVA | | |
|---|---|---|---|---|---|
| | | | Pre_OVA | OVA_2 | OVA_4 |
| Anti-OVA IgG, EU | | | | | |
| A | 10 | 0.0012± 0.0010 | 0.0040± 0.0015 | 0.3531± 0.0497 | 0.6018± 0.0600 |
| B | 10 | 0.0013± 0.0007 | 0.0044± 0.0014 | 0.3691± 0.0390 | 0.6928± 0.0983* |
| C | 10 | 0.0017± 0.0011 | 0.0045± 0.0016 | 0.3588± 0.0427 | 0.7091± 0.0869* |
| D | 10 | 0.0014± 0.0007 | 0.0051± 0.0023 | 0.3699± 0.0452 | 0.7084± 0.0669* |

| Anti-OVA IgM, EU | | | | | |
|---|---|---|---|---|---|
| A | 10 | 0.0444± 0.0137 | 0.0527± 0.0159 | 0.1402± 0.0551 | 0.1404± 0.0334 |
| B | 10 | 0.0471± 0.0178 | 0.0647± 0.0249 | 0.1490± 0.0459 | 0.2254± 0.0764 |
| C | 10 | 0.0450± 0.0139 | 0.0587± 0.0201 | 0.1472± 0.0344 | 0.2655± 0.1472* |
| D | 10 | 0.0432± 0.0204 | 0.0650± 0.0217 | 0.1469± 0.0376 | 0.2571± 0.1665* |

| Anti-OVA IgE, EU | | | | | |
|---|---|---|---|---|---|
| A | 10 | 0.0045± 0.0020 | 0.0110± 0.0023 | 0.0374± 0.0085 | 0.1247± 0.0542 |
| B | 10 | 0.0052± 0.0036 | 0.0128± 0.0037 | 0.0414± 0.0065 | 0.1416± 0.0634 |
| C | 10 | 0.0065± 0.0066 | 0.0114± 0.0031 | 0.0444± 0.0075 | 0.1470± 0.0521 |
| D | 10 | 0.0040± 0.0017 | 0.0110± 0.0015 | 0.0437± 0.0087 | 0.1321± 0.0516 |

| | | | | | |
|---|---|---|---|---|---|
| Anti-OVA immunoglobulin production in serum were measured by ELISA *: indication of statistical significance when compare to negative control EU = (*A*ₛₐₘₚₗₑ - *A*_{blank})/(*A*ₚₒₛᵢₜᵢᵥₑ - Ablank) A: Negative control B: Lower dose C: Higher dose D: Positive control | | | | | |

## Claims

1. An use of the fungal immunomodulatory protein for immunotherapy, wherein the protein having the amino acid sequence of SEQ ID No: 1 
MSDTALIFRLAWDVKKLSFDYTPNWGRGNPNNFIDTVTFPKVLTDKAYTYRV
AVSGRNLGVKPSYAVESDGSQKVNFLEYNSGYGIADTNTIQVFVVDPDTNND
FIIAQWN
 or
SEQ ID No:3

SATSLTFQLAYLVKKIDFDYTPNWGRGTPSSYIDNLTFPKVLTDKKYSYRVVV
NGSDLGVESNFAVTPSGGQTINFLQYNKGYGVADTKTIQVFVVIPDTGNSEEYI
IAEWKKT.

2. The use according to Claim 1, wherein the protein is obtained from *Ganoderma* species or *Flammulina velutipes*.

3. The use according to Claim 1, wherein the *Ganoderma* species is *Ganoderma tsugae*.

4. The use according to Claim 1, wherein the protein is provided from a recombinant *Escherichia coli* or Yeast

5. The use according to Claim 1, which could be applied as adjuvant for alleviating the pain or side effects of a patient suffering cancer.

6. The use according to Claim 1, wherein the immunotherapy is directed to stimulate or activate immunological function.

7. The use according to Claim 6, wherein the immunological function is made by the mechanism selecting from the group consist of activation of natural killer cells, activation of macrophages and multiplication of serum antibodies.

8. The use according to Claim 7, wherein the function is made by activation of natural killer cells and inhibition of telomerase.

9. The use according to Claim 1, wherein the protein is administered orally.

10. The use according to Claim 9, wherein the protein is in the dosage form of solution, sachet, tablet or pellet.
